(19) Europäisches Patentamt
European Patent Office
Office européen des brevets

(11) Veröffentlichungsnummer : **0 490 151 B1**

(12) **EUROPÄISCHE PATENTSCHRIFT**

(45) Veröffentlichungstag der Patentschrift :
**04.05.94 Patentblatt 94/18**

(51) Int. Cl.[5] : **C07C 209/36,** B01J 23/44, B01J 23/46, B01J 23/96

(21) Anmeldenummer : **91120026.9**

(22) Anmeldetag : **25.11.91**

(54) **Verfahren zur Herstellung von Anilin.**

(30) Priorität : **07.12.90 DE 4039026**

(43) Veröffentlichungstag der Anmeldung :
**17.06.92 Patentblatt 92/25**

(45) Bekanntmachung des Hinweises auf die Patenterteilung :
**04.05.94 Patentblatt 94/18**

(84) Benannte Vertragsstaaten :
**BE DE ES FR GB IT NL**

(56) Entgegenhaltungen :
**EP-A- 0 011 090**
**JOURNAL OF THE CHEMICAL SOCIETY, CHEMICAL COMMUNICATIONS. 1981, LETCHWORTH GB Seiten 540 - 541; Savoia, Diego; Trombini, Claudio; Umani-Ronchi, Achille; Verardo, Giancarlo: 'Active metals from potassium-graphite. Palladium-graphite as catalyst in the hydrogenation of nitro compounds, alkenes, and alkynes'**

(73) Patentinhaber : **BAYER AG**
**D-51368 Leverkusen (DE)**

(72) Erfinder : **Immel, Otto, Dr.**
**Immenhofweg 26**
**W-4150 Krefeld (DE)**
Erfinder : **Waldmann, Helmut, Dr.**
**Henry-T.-von-Böttinger-Strasse 15**
**W-5090 Leverkusen 1 (DE)**
Erfinder : **Braden, Rudolf, Dr.**
**Nothauser Feld 1**
**W-5068 Odenthal-Scheuren (DE)**
Erfinder : **Fröhlich, Christian, Dr.**
**Doerperhofstrasse 14**
**W-4150 Krefeld (DE)**
Erfinder : **Friedhofen, Gerhard, Dr.**
**Holzapfelweg 3**
**W-4150 Krefeld (DE)**
Erfinder : **Niemeier, Wilfried, Dr.**
**Minkweg 10b**
**W-4150 Krefeld (DE)**



Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue Saint-Denis, 75001 PARIS

## Beschreibung

Die vorliegende Erfindung betrifft ein verbessertes Verfahren zur Herstellung von Anilin durch katalytische Hydrierung von Nitrobenzol in der Gasphase.

Anilin ist dem Fachmann als wichtiges Zwischenprodukt, beispielsweise zur Herstellung von Farbstoffen und Polyurethanen bekannt.

Zur Hydrierung von Nitrobenzol und anderen Nitroaromaten sind Palladium enthaltende Trägerkatalysatoren bekannt. Entscheidend für die Wirksamkeit eines Katalysators sind neben dem katalytisch aktiven Metall auch die als Katalysatorträger eingesetzten Materialien. Diese Trägermaterialien können aufgrund ihrer Oberflächenbeschaffenheit zur Bildung unerwünschter Nebenprodukte führen. Aus DE-OS 21 35 155 und DE-OS 22 44 401 sind als Träger Aluminiumoxide bekannt, die ganz oder teilweise in Spinell übergeführt worden sind. Weitere Verfahren zur Hydrierung von Nitrobenzol sind aus DE-OS 2 848 978 und DE-OS 2 849 002 bekannt.

Die bisher bekannten Katalysatoren zur Hydrierung von Nitroaromaten in der Gasphase zeigen Nachteile, die ihren Einsatz in der chemischen Technik beschränken. So ist zwar die anfängliche Ausbeute bei der Hydrierung von Nitrobenzol zu Anilin mit 99% recht hoch; die bekannten Katalysatoren verlieren jedoch rasch ihre Aktivität, beispielsweise durch Vergiftung oder durch Abscheiden teeriger Niederschläge, so daß sich nur kurze Standzeiten ergeben. In diesem Fall muß die Hydrierung unterbrochen werden, um den Katalysator zu regenerieren. Die häufigen Unterbrechungen, sowie die aufwendige Art der Regenerierung sind für großtechnische Anlagen unwirtschaftlich. Ein weiterer Nachteil, der bisher bekannten Katalysatoren ist ihre unregelmäßige Aktivität, die sich häufig in einer Überreaktivität zu unerwünschten Nebenprodukten äußert. Wenn man zur Steigerung der Standzeit größere Edelmetallkonzentrationen oder vielfältige Kombinationen unterschiedlicher Elemente anwendet, wirkt sich der große Bedarf an Edelmetallen bei der technischen Realisierung solcher Verfahren als unerwünschter Kostenverursacher aus (DE-OS 28 49 002). Solche aus vielen Elementen hergestellten Katalysatoren sind darüber hinaus umständlicher herzustellen und zu entsorgen.

Aus Journal of the Chemical Society, Chemical Communications, 1981, Seite 540-541 ist bereits eine Palladium-Graphit-Verbindung der Formel $C_{16}Pd$ bekannt, die als Katalysator bei der Hydrierung von Nitrobenzolen in Methanol eingesetzt wird.

In US-A-3.804.916 sind Graphitkatalysatoren beschrieben, bei denen Ni, Pt bzw. Palladium in das Graphitgitter eingebaut sind.

Es wurde gefunden, daß man die Herstellung von Anilin durch katalytische Hydrierung von Nitrobenzol in der Gasphase mit wesentlich verbesserter Katalysatorbelastung bei unerwartet langen Standzeiten durchführen kann, wenn man die Hydrierung in Gegenwart eines Trägerkatalysators durchführt, der Pd oder eine Kombination von Pd mit Ir und/oder Rh und/oder Ru auf Graphit oder graphithaltigem Koks enthält.

Die Erfindung betrifft demnach ein Verfahren zur Herstellung von Anilin durch Hydrierung von Nitrobenzol der Formel

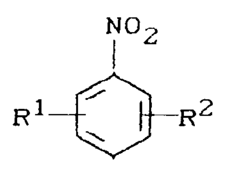 (I),

worin

R¹ und R² unabhängig voneinander für Wasserstoff, Methyl, Ethyl, Propyl oder Butyl, bevorzugt Methyl oder Ethyl, stehen,

in Gegenwart eines edelmetallhaltigen Katalysators in der Gasphase, das dadurch gekennzeichnet ist, daß als Katalysator Palladium auf Graphit oder graphithaltigem Koks als Träger, der eine BET-Oberfläche von 0,2 - 10 $m^2/g$ hat, mit 0,001-1,5 Gew.-% Pd, bezogen auf das Gesamtgewicht des Katalysators, eingesetzt wird, wobei das Pd zu 0 - 40 Relativprozent seiner Menge durch Ir und/oder Rh und/oder Ru ersetzt sein kann.

Die Erfindung betrifft weiterhin einen Katalysator, der auf Graphit oder graphithaltigem Koks als Träger, der eine BET-Oberfläche von 0,2 - 10 $m^2/g$ hat , mit 0,001-1,5 Gew.-% Pd, bezogen auf das Gesamtgewicht des Katalysators, imprägniert ist, wobei das Pd zu 0 - 40 Relativprozent seiner Menge durch Ir und/oder Rh und/oder Ru ersetzt sein kann.

Die erfindungsgemäßen Katalysatoren enthalten also das (die) Edelmetall(e) in folgenden Anordnungen auf dem Träger: Pd allein, Pd/Ir, Pd/Rh, Pd/Ru, Pd/Ir/Rh, Fd/Ir/Ru, Pd/Rh/Ru, Pd/Ir/Rh/Ru. In vielen Fällen wird eine der genannten Zweierkombinationen oder Pd allein eingesetzt.

In bevorzugter Weise liegt in den erfindungsgemäßen Katalysatoren das Palladium in einer Menge von

0,005-1 Gew.-%, bevorzugt 0,05-0,5 Gew.-%, bezogen auf das Gesamtgewicht des Katalysators, vor. Die Untergrenze Null der Relativprozente der anderen genannten Platinmetalle zeigt den Gebrauch von Pd allein an. Falls die anderen Platinmetalle eingesetzt werden, beträgt ihr Anteil bevorzugt insgesamt 10-40 Relativprozent; untereinander beträgt ihr Gewichtsverhältnis 1:1-3:1 zwischen je zweien.

Es hat sich weiterhin als vorteilhaft erwiesen, die genannten Katalysatoren zusätzlich mit einer phosphorhaltigen Verbindung zu dotieren. Ein solcher zusätzlicher Gehalt an Dotierungsmittel beträgt 0,1-2 Gew.-%, bevorzugt 0,1-1 Gew.-% Phosphor als Sauerstoffsäuren des Phosphors $H_3PO_3$, $H_3PO_3$, $H_3PO_2$ oder deren Alkalisalze, wie z.B. Natriumdihydrogenphosphat, Natrium- oder Kaliumphosphat oder Natriumhypophosphit, bezogen auf das Gesamtgewicht der Katalysators.

Die erfindungsgemäßen Katalysatoren sind durch ihren Träger aus einem graphithaltigen Material gekennzeichnet. Solche Materialien sind die Graphite selber und Kokse, wie Nadelkoks oder Petrolkoks. Diese Träger haben eine BET-Oberfläche von 0,2-10 $m^2/g$.

Zur Herstellung der erfindungsgemäßen Katalysatoren kann so vorgegangen werden, daß auf einen der genannten Träger in Form von Pillen, Kugeln, Stranggranulat oder Bruchstücken von etwa 1-10 mm Abmessung die genannten Edelmetalle in Form geeigneter Salze sowie die schwefelhaltige oder phosphorhaltige Verbindung in getrennten Arbeitsgängen aufgetragen werden, wobei nach jedem Auftrag getrocknet wird. Das Trocknen geschieht in bekannter Weise, beispielsweise bei 100-140°C und vermindertem bis normalem Druck, beispielsweise bei 1-1.000 mbar; als verminderter Druck kommt beispielsweise der einer Wasserstrahlpumpe in Betracht. Zum Tränken des Trägers können wäßrige Lösungen verwendet werden. Dies ist in bevorzugter Weise bei den schwefel- oder phosphorhaltigen Verbindungen, von denen wasserlösliche bevorzugt werden, der Fall. Die Edelmetallsalze werden jedoch bevorzugt in organischen Lösungsmitteln, wie einfachen Alkoholen, Ketonen, cyclischen Ethern oder Nitrilen, gelöst und aufgetragen. Beispiele für solche organischen Lösungsmittel sind Methanol, Ethanol, Propanol, Isopropanol, Aceton, Methyl-ethylketon, Dioxan, Acetonitril und vergleichbare Lösungsmittel. Bei Salzen mit organischen Anionen können auch Methylenchlorid und vergleichbare Lösungsmittel eingesetzt werden. Geeignete Salze der Edelmetalle sind beispielsweise ihre Chloride, Nitrate oder Acetate.

Nach dem Imprägnieren und dem abschließenden Trocknen steht der erfindungsgemäße Katalysator zur Verfügung. Er wird in bevorzugter Weise im Reaktor vor Beginn der Hydrierung von Nitrobenzol durch eine Behandlung mit Wasserstoff bei erhöhter Temperatur aktiviert. Eine solche erhöhte Temperatur liegt beispielsweise im Bereich von 200-450°C, bevorzugt im Bereich von 220-420°C.

Die genannten Katalysatoren sind in hervorragender Weise zur Hydrierung von Nitrobenzol zu Anilin verwendbar. Eine solche Hydrierung wird in der Gasphase in einem weiten Druckbereich durchgeführt, der von vermindertem Druck über Normaldruck, nur mäßig erhöhtem Druck bis zu Hochdruck reicht und zahlenmäßig durch den Bereich von 0,5-100 bar ausgedrückt werden kann. Während also sehr wohl bei Hochdruck, beispielsweise 10-100 bar, gearbeitet werden kann, ist jedoch die Variante bei vermindertem Druck, Normaldruck oder nur mäßig erhöhtem Druck bevorzugt. Wegen der Vereinfachung der Apparatur ist das Arbeiten bei Normaldruck besonders bevorzugt. Zur Hydrierung stellt man eine Temperatur von 250-450°C, bevorzugt 350 bis 420°C, ein. Zu dieser Temperatureinstellung wird ein Beitrag von einer äußeren Heizung um den Reaktor oder die Reaktorrohre geleistet, beispielsweise durch einen zirkulierenden Wärmeträger oder angelegte elektrische Heizplatten; der ergänzende Beitrag ist die exotherme Hydrierwärme, die in Abhängigkeit vom Temperaturniveau, vom Substrat und besonders von der Verweilzeit und von der Verdünnung mit $H_2$ einen wechselnden Anteil hat.

Für das erfindungsgemäße Verfahren wird eine Katalysatorbelastung von 0,1-2 kg, bevorzugt 0,2-0,9 kg Nitrobenzol pro Liter Katalysator und Stunde eingestellt.

Der Hydrierwasserstoff wird in einer Menge von 1-100 Normliter pro 1 g eingesetztem Nitrobenzol, bevorzugt 1-50 Normliter, eingesetzt.

Bei Aktivitätsabfall des erfindungsgemäßen Katalysators kann er leicht in situ, d.h. im Hydrierreaktor, regeneriert werden. Hierzu wird der Katalysator bei 350-440°C nacheinander mit Wasserdampf, einem Stickstoff/Luft-Gemisch oder atmosphärischer Luft und schließlich Stickstoff behandelt. Die Behandlung mit Wasserdampf kann 1 bis 3 Stunden, die Behandlung mit Luft bzw. dem Stickstoff/Luft-Gemisch kann 1 bis 4 Stunden erfolgen. Zur erneuten Aktivierung des Katalysators schließt sich eine Behandlung mit Wasserstoff bei 200-350°C an. Eine solche Regenerierung ist nicht möglich bei Edelmetallkatalysatoren auf aktivierter Kohle als Träger, da eine aktivierte Kohle bei einer solchen Regenerierung zu verbrennen beginnt.

Beispiel 1

400 g Petrolkoks-Granulat der Korngröße 2 bis 8 mm und einer BET-Oberfläche von 4,3 $m^2/g$ wurden mit einer Lösung von 1,668 g Pd-Acetat in 72 g Acetonitril getränkt und anschließend bei 100°C getrocknet.

50 g des so mit 0,2 Gew.-% Pd imprägnierten Petrolkoks wurden zusätzlich mit einer Lösung getränkt, die aus 0,029 g $IrCl_4.H_2O$ und 9 g Methanol hergestellt worden war. Das getränkte Granulat wurde 18 Stunden bei 100°C getrocknet. Es enthielt 0,03 Gew.-% Iridium.

Nach dem Trocknen wurden 15 ml (10,1 g) des so hergestellten Katalysators in ein Reaktionsrohr eingefüllt, das einen Innendurchmesser von 17 mm und eine Länge von 60 cm hatte. Das Reaktionsrohr wurde in ein doppelwandiges Stahlrohr geschoben, das senkrecht angeordnet war und mit einem Ölthermostat auf 300°C gehalten wurde. Um den Katalysator zu aktivieren, wurde zunächst 4 Stunden nur Wasserstoff (30 l/h) bei 300°C über den Katalysator geleitet.

Anschließend wurde dem Wasserstoffstrom (30 l/h) noch Nitrobenzol mit dosierter Geschwindigkeit beigemischt und die Temperatur der Ölthermostaten auf 300°C gehalten. Das Reaktionsprodukt hatte in Abhängigkeit von der Betriebsdauer (h) des Katalysators folgende Zusammensetzung (Rest zu 100% sind Nebenprodukte)

| Zeit h | Belastung g/ml.h | Nitrobenzol % | Anilin % |
|---|---|---|---|
| 303 | 0,65 | 0,1 | 99,6 |
| 821 | 0,78 | 0,1 | 99,1 |
| 1485 | 0,67 | 0,6 | 99,1 |
| 1856 | 0,65 | 0,1 | 99,6 |
| 2733 | 0,64 | 0,2 | 99,4 |

Das Reaktionswasser wurde bei der Zusammensetzung des Reaktionsproduktes nicht berücksichtigt,

Beispiel 2

15,2 ml (14,2 g) Graphitgranulat, das mit 0,025% Pd und 0,05% Rh imprägniert worden war, wurde als Katalysator für die Hydrierung von Nitrobenzol benutzt. Nach 4195 Betriebsstunden des Katalysators erbrachte die Hydrierung ein Reaktionsprodukt, das 98,2% Anilin und 1,6% Nitrobenzol enthielt. Dabei betrug die Katalysatorbelastung 0,5 g Nitrobenzol/ml.h. und die Temperatur im Reaktionsofen 300°C. Um den Katalysator zu regenerieren, wurde die Hydrierung unterbrochen und das Reaktionsrohr auf 420°C erwärmt. Es wurden dann 5 Stunden lang 20 l Luft/h und 5 g Wasser/h über die Katalysatorschicht geleitet. Danach wurde der Katalysator erst 1 Stunde bei 300°C mit Wasserstoff (30 l/h) aktiviert, bevor die Hydrierung von Nitrobenzol fortgesetzt wurde. Die Hydrierung erbrachte sodann ein Reaktionsprodukt, in dem 99,4% Anilin, 0,4% Nitrobenzol und 0,2% Nebenprodukte enthalten waren. Das Reaktionswasser wurde analytisch nicht berücksichtigt.

Beispiel 3

100 g Petrolkoks-Granulat der Korngröße 2 bis 8 mm, einer BET-Oberfläche von 4,3 $m^2$ /g und einem Schüttgewicht von 630 g/l wurden mit einer Lösung von 0,417 g Pd-Acetat in 18 ml Acetonitril imprägniert und anschließerid bei 100°C getrocknet. 15 ml des so hergestellten Katalysators wurden wie in Beispiel 1 zur Hydrierung von Nitrobenzol eingesetzt. Der Katalysator, der 0,1 Gew.-% Pd enthielt, wurde zunächst 3,5 Stunden bei 280°C mit Wasserstoff reduziert. Dann wurde dem Wasserstoffstrom noch Nitrobenzol mit dosierter Geschwindigkeit zugemischt und am Katalysator umgesetzt. Im Verlauf von 5737 Stunden wurden 68653 g Nitrobenzol durch die Katalysatorschicht geleitet. Dies entspricht einer Katalysatorbelastung von 0,8 g/ml.h. Bei einer Heiztemperatur von 300° C enthielt das Reaktionsprodukt nach 3891 Stunden 98,9% Anilin und 0,9%

Nitrobenzol und nach 5737 Stunden 98,0% Anilin, 1,7% Nitrobenzol (Rest zu 100 % sind Nebenprodukte). Das Reaktionswasser wurde bei der Zusammensetzung nicht berücksichtigt.

Beispiel 4

100 g Graphitgranulat der Korngröße 2-6 mm mit einer BET-Oberfläche von 0,9 $m^2/g$ wurden mit einer Lösung getränkt, die aus 0,104 g $Pd(CH_3COO)_2$ und 6 g Acetonitril hergestellt wurde. Das imprägnierte Graphitgranulat wurde 18 Stunden bei 100°C getrocknet. 15 ml (14,4 g) des so hergestellten Katalysators wurden wie in Beispiel 1 zur Hydrierung von Nitrobenzol verwendet. Die aufgetränkte Pd-Menge entsprach 0,05 % Pd, bezogen auf das Gesamtgewicht des Katalysators. Um den Katalysator zu aktivieren, wurden zunächst 4 Stunden lang 30 l Wasserstoff pro Stunde bei 280° C über den Katalysator geleitet. Dann wurde mit der Hydrierung von Nitrobenzol begonnen, wobei die Heiztemperatur im Reaktionsofen weiterhin auf 280° C gehalten wurde.

Im Verlauf von 5782 Stunden wurden insgesamt 49906 g Nitrobenzol über den Katalysator geleitet. Das entpricht einer durchschnittlichen Katalysatorbelastung von 0,58 g/ml.h. Nach 3717 h enthielt das Reaktionsprodukt 99,6% Anilin und 0,1% Nitrobenzol; nach einer Versuchsdauer von 5772 Stunden wurden im Reaktionsprodukt 99,5% Anilin und 0,1% Nitrobenzol festgestellt. Der Anteil der Nebenprodukte lag bei 0,3-0,4%.

Beispiel 5

50 g eines handelsüblichen Elektrographits der Korngröße 2-6 mm wurden mit einer Lösung getränkt, die aus 0,1045 g $Pd(CH_3COO)_2$ und 2,3 g Dioxan hergestellt worden war. Das getränkte Graphitgranulat wurde 18 Stunden bei 100°C getrocknet.

30 g des so behandelten Graphitgranulats wurden nochmals mit einer Lösung getränkt, die aus 0,1027 g $NaH_2PO_2.H_2O$ und 1,3 g Methanol hergestellt worden war. Nach dem erneuten Trocknen wurden 10 ml (10 g) zur Hydrierung von Nitrobenzol verwendet. Die aufgetränkte Pd-Menge entsprach 0,1 Gew.-% Pd und die aufgetränkte Natriumhypophosphitlösung entsprach 0,1 Gew.-% P, bezogen auf das Gesamtgewicht des Katalysators. Um den Katalysator zu aktivieren, wurden zunächst 4 Stunden lang 30 l Wasserstoff pro Stunde bei 280° C über den Katalysator geleitet. Dann erfolgte die Hydrierung von Nitrobenzol, wobei die Heiztemperatur im Reaktionsofen weiterhin auf 280° C gehalten wurde. Die Katalysatorbelastung betrug 0,63 bis 0,82 g Nitrobenzol/ml Kat. x h. Nach 1958 Betriebsstunden des Katalysators war noch kein Aktivitätsfall festzustellen.

Das Reaktionsprodukt hatte in Abhängigkeit von der Einsatzdauer des Katalysators folgende Zusammensetzung (Rest zu 100% sind Nebenprodukte):

| Zeit (h) | Belastung g/ml.h | Nitrobenzol (%) | Anilin % |
|---|---|---|---|
| 91 | 0,80 | 0,1 | 99,4 |
| 854 | 0,78 | 0,3 | 99,4 |
| 1361 | 0,81 | 0,4 | 99,4 |
| 1450 | 0,75 | 0,4 | 99,4 |
| 1958 | 0,74 | 0,3 | 99,4 |

Beispiel 6

300 g eines handelsüblichen Nadelkoks der Korngröße 1,5-4 mm mit einer BET-Oberfläche von 0,8-1 $m^2/g$ wurden mit einer Lösung getränkt, die aus 0,625 g Pd-Acetat und 105 g Methylenchlorid hergestellt worden war. Nach einer Zwischentrocknung bei 100° C wurden 100 g des mit Pd imprägnierten Nadelkoks erneut mit einer Lösung getränkt, die aus 0,068 g $NaH_2PO_4.H_2O$ und 35 g Methanol hergestellt worden war. Danach wurde der imprägnierte Nadelkoks 18 Stunden bei 100° C getrocknet.

10 ml (8,5 g) des hergestellten Katalysators, der 0,1 Gew.-% Pd enthielt, wurden in ein Reaktionsrohr eingefüllt, das einen Innendurchmesser von 17 mm und eine Länge von 70 cm hatte. Es wurde in einem senkrecht angeordneten Elektroofen auf 280° C erhitzt. Um den Katalysator zu aktivieren, wurde 1 Stunde lang nur Wasserstoff (30 l/h) durch den Katalysator geleitet. Dann wurden bei einer Heiztemperatur von 280° C Wasserstoff (30 l/h) und Nitrobenzol in dosierter Menge zur Reaktion gebracht. Das Reaktionsprodukt wurde kondensiert und gaschromatographisch analysiert. In Abhängigkeit von der Betriebsdauer des Katalysators, zeigte das Reaktionsprodukt folgende Zusammensetzung (Rest zu 100% sind Nebenprodukte):

| Zeit (h) | Katalysatorbelastung g/ml.h | Nitrobenzol (%) | Anilin % |
|---|---|---|---|
| 117 | 0,75 | 0,1 | 99,2 |
| 487 | 0,82 | 0,1 | 99,3 |
| 726 | 0,96 | 0,1 | 99,5 |
| 919 | 0,69 | 0,1 | 99,5 |
| 1264 | 0,76 | 0,1 | 99,5 |

Beispiel 7 (zum Vergleich)

100 g eines handelsüblichen A-Kohlegranulats, 4 mm Stranggranulat (von Fa. Carbo-Tech, Typ C 40/4) mit einer BET-Oberfläche von 1.200 ± 50 $m^2/g$, wurden mit einer Lösung getränkt, die aus 2,09 g Pd-Acetat und 75 g Acetonitril hergestellt worden war. Das mit Pd imprägnierte A-Kohlegranulat wurde 18 Stunden bei 100° C getrocknet. Die aufgetränkte Pd-Menge entsprach 1 Gew.-% Pd, bezogen auf die eingesetzte A-Kohle. 10 ml (4,3 g) des so hergestellten Katalysators wurden in ein 17 mm weites Glasrohr gefüllt und zunächst 1 Stunde im Wasserstoffstrom (30 l/h) bei 280° C aktiviert. Dabei befand sich das Glasrohr in einem senkrecht angeordneten Elektroofen. Anschließend wurde Nitrobenzol mit einer Geschwindigkeit von 0,71 g/ml Kat. x h über den Katalysator geleitet, wobei der Wasserstoffstrom 30 l/h betrug und die Heiztemperatur im Reaktionsofen auf 280° C gehalten wurde. Das im Verlauf von 162 Stunden entstandene Reaktionsprodukt enthielt

96,2 % Anilin
0,4 % Nitrobenzol
3,4 % Nebenprodukte

Das Reaktionswasser wurde analytisch nicht berücksichtigt.

Beispiel 8

Graphitgranulat (2-8 mm) mit einer BET-Oberfläche von 0,9 $m^2/g$ und A-Kohlegranulat (4 mm Stranggranulat) mit einer BET-Oberfläche von 1200 ± 50 $m^2/g$ wurden in gleicher Weise mit je 0,1 Gew.-% Pd imprägniert. Von beiden Granulaten wurde je 10 g einem offenen Tiegel unter Luftzutritt bei 420° C jeweils 3 Stunden getempert. Danach wurden folgende Gewichtsverluste ermittelt

Graphit (0,1% Pd): 0,22%
A-Kohle (0,1% Pd): 10,1 %

Bei einer Desaktivierung eines Pd-Graphit- bzw. eines Pd-A-Kohle-Katalysators ist eine derartige Temperung bei 380-450° C erforderlich, um eine Regenerierung zu erreichen. Der Vergleichsversuch zeigt, daß die Temperung zur Regenerierung des Pd-A-Kohle-Katalysators mit zu hohen Verlusten verbunden ist.

**Patentansprüche**

1. Verfahren zur Herstellung von Anilin durch Hydrierung von Nitrobenzol der Formel

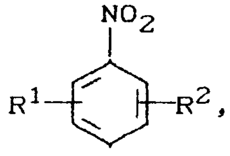

worin

$R^1$ und $R^2$ unabhängig voneinander für Wasserstoff, Methyl, Ethyl, Propyl oder Butyl, bevorzugt Methyl oder Ethyl, stehen,

in Gegenwart eines edelmetallhaltigen Katalysators in der Gasphase, dadurch gekennzeichnet, daß als Katalysator Palladium auf Graphit oder graphithaltigem Koks als Träger, der eine BET-Oberfläche von 0,2 - 10 $m^2/g$ hat, mit 0,001-1,5 Gew.-% Pd, bezogen auf das Gesamtgewicht des Katalysators, eingesetzt wird, wobei das Pd zu 0-40 Relativprozent seiner Menge durch Ir und/oder Rh und/oder Ru ersetzt sein kann.

2. Katalysator, der auf Graphit oder graphithaltigem Koks als Träger, der eine BET-Oberfläche von 0,2 - 10 $m^2/g$ hat, mit 0,001-1,5 Gew.-% Pd, bezogen auf das Gesamtgewicht des Katalysators, imprägniert ist, wobei das Pd zu 0-40 Relativprozent seiner Menge durch Ir und/oder Rh und/oder Ru ersetzt sein kann.

3. Katalysator nach Anspruch 2, gekennzeichnet durch einen Gehalt an Pd in einer Menge von 0,005-Gew.-%, bevorzugt 0,05-0,5 Gew.-%, wobei das Pd zu 0-40 Relativprozent, bevorzugt 10-40 Relativprozent durch Ir und/oder Rh und/oder Ru ersetzt sein kann.

4. Katalysator nach Anspruch 2, gekennzeichnet durch einen zusätzlichen Gehalt von 0,1-2 Gew.-%, bevorzugt 0,1-1 Gew.-%,gerechnet als Phosphor und bezogen auf das Gesamtgewicht des Katalysators, an einer Sauerstoffsäure des Phosphors oder deren Alkalisalze.

5. Verfahren nach Anspruch 1, dadurch gekennzeichnet, daß bei einem Druck von 0,5-100 bar und einer Temperatur von 250-450° C, bevorzugt 350-420° C, gearbeitet wird.

6. Verfahren nach Anspruch 5, dadurch gekennzeichnet, daß bei Normaldruck gearbeitet wird.

7. Verfahren nach Anspruch 1, dadurch gekennzeichnet, daß eine Katalysatorbelastung von 0,1-2 kg, bevorzugt 0,2-1,0 kg Nitrobenzol pro Liter Katalysator pro Stunde eingestellt wird.

8. Verfahren nach Anspruch 1, dadurch gekennzeichnet, daß 1-100 Normliter $H_2$, bevorzugt 1-50 Normliter $H_2$ pro 1 g Nitrobenzol, eingesetzt werden.

9. Verfahren nach Anspruch 1, dadurch gekennzeichnet, daß der Katalysator bei Aktivitätsabfall durch aufeinander folgende Behandlung mit Wasserdampf, einem $N_2$/Luft-Gemisch oder atmosphärischer Luft und $N_2$ bei 350-440° C regeneriert wird.

## Claims

1. A process for the production of aniline by hydrogenation of nitrobenzene corresponding to the following formula:

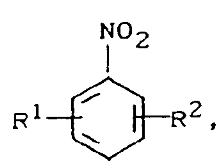

in which

$R^1$ and $R^2$ independently of one another represent hydrogen, methyl, ethyl, propyl or butyl, preferably methyl or ethyl,

in the presence of a catalyst containing noble metals in the gas phase, characterized in that the catalyst used is palladium on graphite or graphite-containing coke with a BET surface of 0.2 to 10 $m^2/g$ as support impregnated with 0.001 to 1.5% by weight Pd, based on the total weight of the catalyst, 0 to 40 relative percent of the Pd being replaceable by Ir and/or Rh and/or Ru.

2. A catalyst impregnated with 0.001 to 1.5% by weight Pd, based on the total weight of the catalyst, on graphite or graphite-containing coke with a BET surface of 0.2 to 10 $m^2/g$ as support, 0 to 40 relative percent of the Pd being replaceable by Ir and/or Rh and/or Ru.

3. A catalyst as claimed in claim 2, characterized by a Pd content of 0.005 to 1% by weight and preferably 0.05 to 0.5% by weight, 0 to 40 relative percent of the Pd being replaceable by Ir and/or Rh and/or Ru.

4. A catalyst as claimed in claim 2, characterized by an additional content of 0.1 to 2% by weight and preferably 0.01 to 1% by weight, expressed as phosphorus and based on the total weight of the catalyst, of an oxyacid of phosphorus or alkali metal salts thereof.

5. A process as claimed in claim 1, characterized in that it is carried out under a pressure of 0.5 to 100 bar and at a temperature of 250 to 450°C and preferably at a temperature of 350 to 420°C.

6. A process as claimed in claim 5, characterized in that it is carried out under normal pressure.

7. A process as claimed in claim 1, characterized in that a catalyst load of 0.1 to 2 kg and preferably 0.2 to 0.1 kg nitrobenzene per litre catalyst per hour is adjusted.

8. A process as claimed in claim 1, characterized in that 1 to 100 standard litres $H_2$ and preferably 1 to 50 standard litres $H_2$ per 1 g nitrobenzene are used.

9. A process as claimed in claim 1, characterized in that, in the event of a loss of activity, the catalyst is regenerated by successive treatment with steam, an $N_2$/air mixture or atmospheric air and $N_2$ at 350 to 440°C.

**Revendications**

1. Procédé pour préparer de l'aniline par hydrogénation du nitrobenzène de formule :

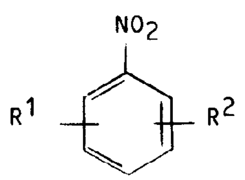

(I),

dans laquelle

$R^1$ et $R^2$ représentent chacun, indépendamment l'un de l'autre, un atome d'hydrogène, un groupe méthyle, éthyle, propyle ou benzyle, de préférence un groupe méthyle ou éthyle,

en présence d'un catalyseur contenant un métal noble en phase gazeuse, procédé caractérisé en ce qu'on utilise comme catalyseur du palladium sur du graphite ou sur du coke contenant du graphite comme support, présentant une surface BET de 0,2 à 10 $m^2/g$, avec 0,001 à 1,5% en poids de Pd, par rapport au poids total du catalyseur, le Pd pouvant être remplacé, pour 0 à 40% de sa quantité relative, par Ir et/ou par Rh et/ou par Ru.

2. Catalyseur qui a été fixé par imprégnation sur du graphite ou sur du coke contenant du graphite comme support, ayant une surface BET de 0,2 à 10 $m^2/g$ et comportant 0,001 à 1,5% en poids de Pd, par rapport au poids total du catalyseur, le Pd pouvant être remplacé, pour 0 à 40% relatif de sa quantité, par Ir et/ou par Rh et/ou par Ru.

3. Catalyseur selon la revendication 2, caractérisé par une teneur en Pd en une quantité de 0,005 à 1% en poids, de préférence 0,05 à 0,5% en poids, le Pd pouvant être remplacé pour 0 à 40% relatifs, de préférence 10 à 40% relatifs, par Ir et/ou par Rh et/ou par Ru.

4. Catalyseur selon la revendication 2, caractérisé par une teneur supplémentaire en 0,1 à 2% en poids, de préférence 0,1 à 1% en poids, calculé en phosphore et par rapport au poids total du catalyseur, d'un acide oxygéné du phosphore ou de ses sels alcalins.

5. Procédé selon la revendication 1, caractérisé en ce qu'on travaille sous une pression de 0,5 à 100 bar et à une température de 250 à 450°C, de préférence 350 à 420°C.

6. Procédé selon la revendication 5, caractérisé en ce qu'on travaille sous la pression normale.

7. Procédé selon la revendication 1, caractérisé en ce qu'on ajuste à une charge de catalyseur de 0,1 à 2 kg, de préférence 0,2 à 1,0 kg de nitrobenzène par litre de catalyseur par heure.

8. Procédé selon la revendication 1, caractérisé en ce qu'on utilise 1 à 100 l normaux de $H_2$, de préférence 1 à 50 l normaux de $H_2$ pour 1 g de nitrobenzène.

9. Procédé selon la revendication 1, caractérisé en ce qu'en cas de chute d'activité, on régénère le catalyseur par les traitements successifs suivants par de la vapeur d'eau, par un mélange $N_2$/air ou par de l'air atmosphérique et par $N_2$ à 350-440°C.